Europäisches Patentamt

European Patent Office

Office européen des brevets

(19)

(11) Publication number: **0 040 862**
A1

## EUROPEAN PATENT APPLICATION

(21) Application number: **81104586.3**

(22) Date of filing: **04.01.80**

(51) Int. Cl.³: **A 61 K 9/70**, A 61 L 15/01,
A 61 L 15/03

(30) Priority: **11.01.79 US 2565**
**11.06.79 US 47084**
**14.08.79 JP 103495/79**

(43) Date of publication of application: **02.12.81**
**Bulletin 81/48**

(84) Designated Contracting States: **AT BE CH DE FR GB IT**
**LU NL SE**

(60) Publication number of the earlier application in
accordance with Art. 76 EPC: **0013606**

(71) Applicant: **Key Pharmaceuticals, Inc., 50 N.W. 176th**
**Street, Miami, Florida 33169 (US)**

(72) Inventor: **Keith, Alec D. Pennsylvania State University,**
**Biophysics Department G18 Life-Sciences Laboratory,**
**University Park, PA 16802 (US)**
Inventor: **Snipes, Wallace Pennsylvania State University,**
**Biophysics Department G18 Life-Sciences Laboratory,**
**University Park, PA 16802 (US)**

(74) Representative: **Laredo, Jack Joseph, Elkington and Fife**
**High Holborn House 52/54 High Holborn, London,**
**WC1V 6SH (GB)**

(54) Burn matrix, method of its preparation and delivery device comprising said matrix.

(57) This invention provides a burn matrix comprising glycerol, polyvinylalcohol, a water-soluble polymer having hydration sites which in combination with the remaining ingredients yields a matrix capable of sustained release of a drug dispersed therein, and water. The matrix may further include a drug for topical or transdermal application to a patient. The invention also provides a method of making the matrix and a delivery device incorporating the matrix. The matrix may be used in the form of a package, which comprises the matrix (14) embedded in a backing member (10) provided with a detachable cover layer (12). Before use, the cover layer is removed, and the backing member applied to the patient by means of a pressure-sensitive adhesive layer (26), so that the matrix (14) is held against the skin of the patient.

0040862

- 1 -

Burn Matrix, Method of its preparation and delivery
device comprising said matrix

This invention relates to a polymeric diffusion
matrix, and is concerned with a burn matrix for application to burn patients.

The matrix comprises, on a weight basis, from
about 2 to about 60 % of glycerol, from about 2 to about
15 % of polyvinylalcohol, and from about 2 to about 10 %
of a water-soluble polymer with hydration sites, e.g.
polyvinylpyrrolidone, the balance being essentially
water. The water-soluble polymer having hydration sites
is compatible with the remainder of the ingredients of
the matrix of the invention to permit the sustained
release of a drug. Other examples of such water-soluble polymers include agar, agarose, and water-soluble
cellulose derivatives. A therapeutically effective
amount of a drug suitable for topical or transdermal
application to a patient may be included in the matrix
to form a drug delivery matrix.

Preferably, the polyvinylalcohol has a molecular
weight of at least about 70,000. In a preferred embodiment, the polyvinylalcohol has a molecular weight of
from about 100,000 to about 150,000. Polyvinylpyrrolidone
is preferred as the water-soluble polymer. The polyvinylpyrrolidone molecular weight should be within
the range of about 20,000 to about 60,000, preferably
from about 35,000 to about 50,000.

In accordance with the invention, there is provided
a polymeric diffusion matrix for application to burned
or wounded areas of a patient. A drug can be dispersed
or deposited on the diffusion matrix for application to
the patient. A drug delivery system suitable for the
transdermal application of a drug to a patient over a
prolonged period at a relatively constant level com-

- 2 -

prises the polymeric diffusion matrix and means for securing the matrix to the skin of a patient.

According to the present invention, a polymeric matrix for application to a burned portion of a patient's body is provided (burn matrix). In this embodiment, the matrix, preferably in a cured state, comprises a water-soluble polymer with hydration sites, polyvinylalcohol, glycerol and water. Examples of the water-soluble polymer include polyvinylpyrrolidone, agar, agarose, water-soluble cellulose derivatives, and other compounds which are compatible with the remainder of the ingredients of the diffusion matrix of the invention to permit sustained release of a drug.

For a matrix in an uncured state, the water-soluble polymer is present in an amount of from about 2 to about 10 %, preferably from about 3 to about 8 % by weight. For polyvinylpyrrolidone, which is a preferred water-soluble polymer, it has a molecular weight of from about 25,000 to about 60,000, preferably from about 35,000 to about 50,000. The polyvinylalcohol is present in an amount of from about 2 to about 15 % preferably from about 6 to about 12 % by weight. The polyvinylalcohol has a molecular weight of from about 100,000 to about 150,000, preferably from about 120,000 to about 135,000. The glycerol is present in an amount of from about 2 to about 20 %, preferably from about 2 to about 18 % by weight. Preferably, the glycerol is a 96 % aqueous glycerol solution.

As a preferred embodiment, there is provided a burn matrix which comprises in its uncured state and on a weight basis: about 10.5 % of polyvinylalcohol (molecular weight 126,000); about 6 % of polyvinylpyrrilidone (molecular weight 40,000); about 15 % of glycerol; and the balance water.

The relative weight amounts of polyvinylalcohol to polyvinylpyrrolidone that have been considered range from about 3:1 to about 1:1. In actual practice, however, at a range of about 3:1, less than optimum results are obtained with the burn matrix swelling to an unacceptable degree, and at the ratio of 1:1 the burn matrix tends toward being soft and sticky. Thus, in accordance with a preferred aspect of the present invention, we have found that a weight range of polyvinylalcohol to polyvinylpyrrolidone should be between about 2:1 and about 3:2. The weight ratio of glycerol to total polymers for the burn matrix is usually less than 1, preferably about 0.5-1:1.

The amount of water which is to be utilized in the preparation of a burn matrix in accordance with the present invention in its generic aspect is related to the amount of glycerol which is used to make the burn matrix of the present invention. The amount of water by volume exceeds the amount of glycerol that is used in the initial mixture of ingredients. According to a preferred embodiment of the present invention, water is present in an amount of from about three to about seven times the amount of glycerol present in the initial mixture of ingredients. After the manufacture of the burn matrix of the present invention, the matrix is "cured" to eliminate most of the water, where water has been used in excess. The amount of time for the cure depends upon conditions such as the amount of excess water. In a preferred embodiment where 20 ml of glycerol is mixed with 100 ml of water, the cure time is about 24 hours, yielding a burn matrix with an

0040862

approximately equal amount of water and glycerol.

In order to prepare the burn matrix of the present invention, the water and glycerol are mixed together, preferably at a somewhat elevated temperature, e.g. 50°C. The polyvinylalcohol and the polyvinylpyrrolidone are added under agitation with the temperature being raised and with continued agitation until solution is effected. The temperature in one embodiment is raised to about 95°C with solution being effected at that temperature. The resulting homogeneous mixture is then poured onto forms which are typically of glass or stainless steel serving as templates to produce a burn matrix having a thickness of about 3 to about 4 mm. Where excess water has been included in the burn matrix, the burn matrix is cured to permit elimination of the excess water. For example, where a 5:1 volume ratio of water to glycerol was used, the freshly prepared burn matrix was permitted to set for about 24 hours, resulting in a burn matrix having a thickness of about 1 to 2 mm. The preferred thickness for a "cured" burn matrix is from about 0.1 to about 2 mm.

The molten burn matrix is preferably cast to form a sheet of the matrix. After curing, the sheet is cut into smaller sheets having a suitable surface area. The smaller sheets can then be deposited on an appropriate backing layer. Alternatively, the molten burn matrix material can be poured onto a backing layer to form a sheet of the matrix in intimate contact with the backing layer. The backing layer can be made of laminates comprising a polyester outer layer, a metal foil intermediate layer, and a ionomer inner layer. The matrix/backing layer laminate can be wound to form a roll of the matrix or cut into smaller sheets of suitable size.

Where drugs are to be included in the burn matrix,

they may be added, in the case of drugs soluble in the burn matrix, to the homogeneous mixture prior to casting, or after curing of the matrix, by the physician or pharmacist at his direction shortly before the need for application arises, permitting a wider flexibility in topically applying a medicine to the patient. Generally, water-insoluble drugs may be included in the burn matrix either through original incorporation into the mixture of water and glycerol or through subsequent application of the drug into the already prepared burn matrix. Where the drug is to be applied to a typical burn matrix of the invention having a thickness of about 2 mm, the drug may be painted onto the surface of the burn matrix or it may be applied through other means, such as an aerosol. A sufficient period of time, e.g., 4 hours, should be provided for the drug to diffuse through the burn matrix of the present invention. In order to provide an anesthetic effect, a water-soluble anesthetic such as xylocaine may be applied through any of the above modes available for water-soluble drugs. The amount of the water-soluble drug that is to be dispersed in the burn matrix of the present invention should be in excess of the amount which is to be administered to the patient. An excess of 1:1 to 10 times the actual amount of drug which is to be administered to the patient should generally be used.

A water-soluble antibiotic to counter the possibility of infection should also be considered for inclusion in the burn matrix of the present invention. Because of the option of including the specifically desired antibiotic after the preparation of the burn matrix of the present invention, the individual physician is given great latitude in selecting the desired antibiotic to take into account the particular needs of the specific patient being treated. An example of a water-soluble antibiotic which may be incorporated into the burn matrix of the present invention is Penicillin VK.

Water-insoluble materials may also be desirably included in the burn matrix of the present invention. Such materials are preferably introduced directly into the intitial mixture of water and glycerol at the outset of the manufacturing process for making the burn matrix of the present invention. In accordance with one aspect of the invention, there is provided a zinc substance in an amount from about 0.4 to about 2 % by weight based upon the final weight of the cured burn matrix of the present invention. Zinc chelates may be used as the zinc substance of this aspect of the present invention. In accordance with a further embodiment within the scope of the present invention, about 0.4 to about 2 % by weight of zinc or silver sulphadiazine is incorporated into the burn matrix of the present invention for retarding Pseudomonas infections.

In making a zinc or silver ( or other water-insoluble containing) burn matrix of the present invention, the zinc or silver material is preferably added with a small amount of the glycerol. The amount of glycerol needed to make the suspension is substrated from the amount of glycerol initially mixed with the water. A uniform suspension of the zinc or silver compound and glycerol is added together with the water and remainder of the glycerol, preferably as the last stage prior to casting.

In addition to local anesthetics and antibiotics which can be applied to or incorporated into the burn matrix of the present invention, other topical medicines may also be applied to or incorporated in the burn matrix. Examples of useful topical medicines include fungicides, bactericides, anti-micoplasma (E. coliplasma), analgesics and local anesthetics.

The amount of drug which can be incorporated into

- 7 -

the burn matrix is up to about 1 % by weight of the burn matrix. The term "incorporated" means that the drug is added to the polymer mixture before casting. The amount of drug which can be painted onto the surface of the matrix varies in accordance with the drug applied.

A hydrophobic casting may be desired in the burn matrix of the present invention. For this purpose, silicone oil may be added in amounts of about 0.1 to 10 % by weight, based on the matrix in the initial mixture of glycerol and water. Mineral oil or vegetable oil may substitute in part or whole for the silicone oil, which lowers the transdermal loss of water in the patient.

The burn matrix in accordance with the present invention is a flexible and transparent polymer which is suited for being applied directly to a burned portion of the patient being treated for most parts of the body. After hydration, the burn matrix is highly flexible and will adhere mildly to the skin. The degree of adherence is sufficient to hold the burn matrix in place but not enough to injure the patient's skin when it is removed. It is contemplated that the burn matrix should be replaced periodically, typically at 24 hour or longer intervals.

The burn matrix of the present invention may be stored for prolonged periods, particularly when placed in a sealed container.

- 8 -

The water-soluble polymer complements the polyvinylalcohol by providing retention of shape of the desired diffusion matrix. Examples of such a water-soluble polymer with hydration sites suitable for the present invention include agar, agarose, polyvinylpyrrolidone and water-soluble cellulose derivatives. The matrix may further contain a therapeutically effective amount of one or more drugs suitable for topical or transdermal application to a patient.

The present polymeric diffusion matrix may be used in the uncured or in the cured state. It may be used as a burn matrix without any drug, although this is not preferred. The term "cured" means that the polymeric diffusion matrix contains little or no excess water used in forming the matrix. As shown below, the diffusion matrix is formed by mixing together the glycerol, polyvinylalcohol, the water-soluble polymer with hydration sites and water to obtain a homogenous mixture which is

- 9 -

cast into sheets of the matrix. In order to allow casting of the mixture, it is sometimes necessary to use an excess amount of water. Immediately after casting, the polymeric matrix is in an "uncured"state. The excess water may then be permitted to evaporate. When substantially all of the excess water has evaporated, the polymeric matrix is in a "cured" state. As a result of the evaporation of the water, which generally requires from about 1 to about 18 hours, the thickness of the diffusion matrix is reduced, or a "collapsed" matrix is obtained.

The diffusion matrix of the present invention provides a steady release of the drug to the patient over an extended period, typically 24 hours.

In its uncured state, the polymeric diffusion matrix comprises, preferably, from about 2 to about 20 % of glycerol, from about 2 to about 15 % of polyvinylalcohol, from about 2 to about 10 % of polyvinylpyrrolidone, and the balance water, all percentages being by weight. Agar, agarose, water-soluble cellulose derivatives or other compatible substances may replace all or part of the polyvinylpyrrolidone.

In the uncured matrix, the glycerol is present in an amount of from about 2 to 60 %, preferably 2 to 20 %, by weight. However, when trinitroglycerol is the drug to be applied, the amount of glycerol should be within the range of from about 35 to 60 %. Preferably, the glycerol has a minimum specific gravity of 1.23 g/ml.

The polyvinylalcohol is present in the uncured matrix in an amount of from about 2 to about 15 %, preferably from about 4 to about 9 % by weight. Preferably,

- 10 -

the polyvinylalcohol has a molecular weight of at least about 70,000. Most preferably, the molecular weight is from about 100,000 to about 150,000.

The water-soluble polymer with hydration sites is present in the uncured matrix in an amount of from about 2 to about 10 %, preferably from about 2 to about 5 %, by weight. In a preferred embodiment, polyvinylpyrrolidone is used as the water-soluble polymer. The molecular weight for the polyvinylpyrrolidone should be selected to maintain water solubility. In general, this molecular weight should be within the range of from about 20,000 to about 60,000, preferably from about 35,000 to about 50,000. The polyvinylpyrrolidone may be replaced by other ingredients which permit sustained release. For example, agar in an amount of from about 2 % to about 6 % by weight may be used.

The balance of the matrix comprises essentially water.

When the matrix is uncured, it preferably comprises from about 2 to about 20 % of glycerol, from about 6 to about 12 % of polyvinylalcohol, from about 3 to about 8 % of the water-soluble polymer having hydration sites, and the balance water.

In its cured state, the polymeric diffusion matrix comprises from about 2 to about 55 %, preferably from about 4 to about 35 % of glycerol, from about 4 to about 30 %, preferably from about 8 to about 20 % of polyvinylalcohol; from about 2 to about 20 %, preferably from about 4 to about 10 % of a water-soluble polymer having hydration sites, preferably polyvinylpyrrolidone, and the balance water, all percentages being by weight. The molecular weight ranges for the polyvinylalcohol and polyvinylpyrrolidone are the same for cured and uncured diffusion matrices. The cured matrix has a density of about 1.2 g/ml. It is noted that the weight ratio of glycerol to water in the

- 11 -

cured matrix is about 0.6 - 1.8:1, preferably about 1:1. The cured matrix shows little swelling when immersed in water and will not dissolve in water at room temperature. However, if the water is heated to boiling, the diffusion matrix will dissolve.

At least one drug may be dispersed throughout the diffusion matrix. The type of drug which may be dispersed in the diffusion matrix of the present invention includes any drug which is capable of being transdermally or topically administered to a patient. With the sustained release of the drug at a relatively steady rate over a prolonged period, typically 24 hours, the patient is provided with the benefit of a steady application of the drug over the prolonged period. Examples of drugs which are suitable for inclusion in the diffusion matrix of the present invention include the following: alpha-[1(methylamino)-ethyl]-benzene methanol, which is useful as an adrenergic (bronchodilator); N-phenyl-N-[1-(2-phenylethyl)-4-piperidinyl propanamide, useful as a narcotic analgesic; 6-chloro-3,4-dihydro-2H-1,2,4-benzothiadiazine-7-sulphonamide 1,1-dioxide, useful as a diuretic; 2-diphenylmethoxy-N,N-dimethylethanamine, useful as an antihistamine; and an estrogen. Other useful drugs include: anti-microbial agents such as penicillin, tetracycline, oxytetracycline, chlortetracycline, chloramphenicol, and sulphonamides; sedatives and hypnotics such as pentabarbital sodium, phenobarbital, secobarbital sodium, codeine, ( -bromoisovaleryl)urea, carbromal, and sodium phenobarbital; psychic energizers such as 3-(2-aminopropyl)indole acetate and 3-(2-aminobutyl).indole acetate; tranquilizers such as reserpine, chlorpromazine hydrochloride, and thiopropazate hydrochloride; hormones such as adrenocortisosteriods, for example, 6-methylprednisolone; androgenic steroids, for example, methylestosterone, and fluoxymesterone;

- 12-

estrogenic steroids, for example, estrone, estradiol and ethinyl estradiol; progestational steroids, for example, 17-hydroxyprogesterone acetate, medroxyprogesterone acetate, 19-norprogesterone, and norethindrone; and thyroxine; antipyretics such as aspirin, salicylamide, and sodium salicylate; morphine and other narcotic analgesics; antidiabetics, e.g. insulin; antispasmodics such as atropine, methscopolamine bromide, methscopolamine bromide with phenobarbital; antimalarials such as the 4-aminoquinolines, 9-aminoquinolines, and pyrimethamine; and nutritional agents such as vitamins, essential amino acids, and essential fats. The above listing of drugs is merely exemplary of the transdermally applicable drugs. It is contemplated that any drug which may be transdermally applied is suitable for use as the drug to be applied via the diffusion matrix in the present device.

It will be appreciated that the drug may be added to the above mixture not only in the form of the pure chemical compound, but also in admixture with other drugs which may be transdermally applied or with other ingredients which are not incompatible with the desired objective of transdermally administering the drug to a patient. Thus, simple pharmacologically acceptable derivatives of the drugs such as ethers, esters, amides, acetals, salts and the like may be used. In some cases such derivatives may actually be preferred.

The amount of the drug dispersed in the diffusion matrix can be varied in accordance with the desired dosage and the length of time the matrix is to remain on the skin. However, the amount of the drug included in the matrix should generally be in excess of the amount which is to be delivered to the patient. If the diffusion matrix is to be used for 24 hours, an approximate 10-fold excess of the drug should be included.

- 13 -

The following methods have been found convenient for preparing the diffusion matrix of the present invention.

In a first method, the matrix is formed at atmospheric pressure. Water and glycerol are first mixed together. Since it has been found that alkaline mixtures have relatively poor stability, the pH of the mixture is adjusted so that it is either neutral or slightly acidic, i.e., the pH ranging from about 6.5 to about 7.0. In a preferred embodiment, the pH is adjusted to within the above-mentioned range by adding sodium citrate and citric acid to the mixture.

The polyvinylalcohol and polyvinylpyrrolidone are then added to the glycerol-water mixture at room temperature, with agitation. The mixture is heated to a temperature within the range of from about 90 to about 95°C at atmospheric pressure to extend the polymers. The mixture is held at this temperature for about one hour. If desired, the mixture may be maintained at this temperature for a period of about 48 hours prior to the addition of the drug. That is, the mixture is stable for a period of about 48 hours and may be kept for such a period before being mixed with the drug to be delivered to the patient. Thereafter, the mixture is cooled to 80°C and stirred for an additional hour to remove bubbles therefrom. The drug to be applied to the patient is then added to the mixture, with thorough agitation. Once a homogeneous mixture of the polymer solution and drug is obtained, the mixture is ready to be cast into sheets of the drug-containing diffusion matrix. In a preferred embodiment, the drug may be dissolved by agitation in a suitable solvent such as glycerin and water. The solution obtained in this way can be maintained at room temperature for prolonged periods without deterioration.

In a second method, water and glycerol are mixed, with the pH of the mixture adjusted to a desired value by adding suitable amounts of sodium dictrate and citric acid. Thereafter, the polyvinylalcohol and polyvinylpyrrolidone are added. The resulting mixture is then heated to a temperature of about 120°C at a pressure of about 2 atmospheres absolute. The temperature is maintained for about 1 hour without any mechanical agitation. In a preferred embodiment, the heating may be performed in an autoclave. Since bubbles are not formed when the heating is conducted in an autoclave, such a procedure is preferred. Thereafter, the temperature is lowered to about 20 to about 80°C whereupon the drug to be applied to the patient is added. After the drug has

- 15 -

been homogeneously dispersed in the liquid mixture, the mixture is poured into moulds to form sheets of the drug-containing diffusion matrix.

In the above methods, the drug must be added and mixed thoroughly when the polymer mixture is in the liquid state. Furthermore, the mixture should be cast within about 30 minutes after the drug has been introduced into the polymer solution. This is important in order to avoid the setting of the polymer solution prior to casting.

The temperature at which the drug is to be added to the matrix solution depends on the stability of the drug.

A hydrophobic coating on a drug delivery matrix= is desired for treating patients having wounds or burns. Silicone oil may be added in amounts of about 0.1 to 10 % by weight, based on the matrix, in the initial mixture of glycerol and water. Mineral oil or vegetable oil may be substituted in whole or in part for the silicone oil. The oil serves to reduce transdermal loss of water in the wounded or burned patient.

Dodecyl alcohol of sorbitan (Tween-20) or other detergents may be added in an amount of 0.1 to 10 % by weight, based on the matrix, as a dispersing agent, if desired.

For drugs that are alcohol-soluble, it may be desirable to add ethanol or isopropanol in an amount of from 2 to 40 % by weight, based on the matrix, in the initial mixture of glycerol and water, to facilitate the preparation of a diffusion matrix for such alcohol-soluble drugs. In addition, ethanol and isopropanol, when added to the initial mixture, will provide a "collapsed" diffusion matrix, i.e. as the ethanol and isopropanol evaporate the diffusion matrix produced in accordance with the present invention will "collapse".

An absorption facilitator to ensure skin penetration such as dimethylsulphoxide, decylmethylsulphoxide, or other penetration enhancers may be added.

If it is desired to increase the effective lifetime of the diffusion matrix, a drug reservoir may also be attached to the diffusion matrix.

The present drug delivery device comprises the drug-containing diffusion matrix and means for fastening the matrix to the skin of a patient. Such means can take various forms, such as an occlusive backing layer forming a kind of "bandage" with the diffusion matrix being held against the skin of a patient being treated. A polyethylene or Mylar tape is contemplated as one form of occlusive layer in accordance with the present invention. It can also take the form of an elastic band, such as a cloth band, a rubbery band or other material. Here, the diffusion matrix is placed directly on the skin and held in place by such elastic band which typically will be placed over the arm or wrist of the patient. An intermediate adhesive layer between the diffusion matrix and the skin capable of permitting the transdermal application of the drug can also be used.

As a preferred embodiment in the packaging of the present matrix, the drug-containing diffusion matrix is placed in a cavity provided in an inert backing material. Useful backing materials include metal foils such as aluminium foil, polyolefins such as polyethylene and polypropylene, polyesters such as Mylar (polyethylene terephthalate), polyamides such as Nylon and the like. The drug-containing diffusion matrix can be poured in its molten state into the cavity and permitted to cool. An adhesive layer is provided on the backing material surrounding the cavity. To prevent air from coming into contact with the matrix, the adhesive layer and the matrix are sealed with a release layer. To use the device, the patient peels off the release layer and places the device in intimate contact with his skin. The exposed adhesive layer secures the device to the patient. Since a concentration gradient exists in a plane normal to the surface of the matrix and the patient's skin, this condition facilitates the diffusion of the drug through the matrix into the patient's body. Thus, there is provided a device whereby a drug is delivered transdermally to a patient at a steady rate over a prolonged period of time.

Reference is now made to the accompanying drawings, in which:

Figure 1 shows a plan view of a bandage having incorporated therein the drug-containing polymeric diffusion matrix of the present invention; and

Figure 2 illustrates the cross-sectional view along line 2-2' in Figure 1.

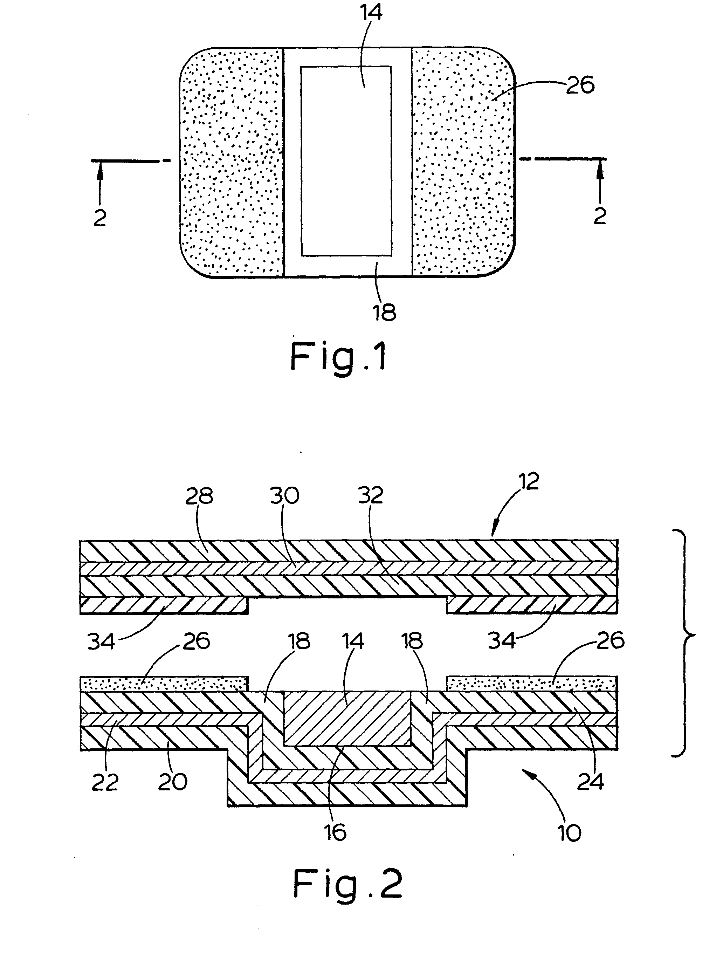

The construction of a preferred embodiment for the packaging of the present invention is shown in further detail in Figures 1 and 2. As illustrated in the Figures, the package comprises a bandage having a cover layer 12 and a backing member 10. The diffusion matrix 14 having a drug                    dispersed therein is placed in cavity 16 in the backing member 10. The diffusion matrix may be poured in its molten state into the cavity 16 in the backing member 10 and permitted to cure. Alternatively, the molten polymeric mixture (with or without a drug) is cast to form a thin sheet which is cut, after curing, into smaller sheets to fit the particular application of the matrix. Individual smaller sheets may then be placed in the cavity 16 in the backing member 10. The area 18 surrounding the matrix in the backing member 10 is heat-sealed to prevent the matrix from being removed from the backing member. The backing member 10 is formed of a laminate comprising an outer layer 20 made of a polyester, such as polyethylene terephthalate, an intermediate layer 22 made of a metallic foil, e.g. aluminium foil, and an inner layer 24 made of an ionomer, such as Surlyn. A layer of pressure sensitive adhesive 26 is provided on the surface of the inner layer surrounding the heat sealed portion. It is noted that the adhesive does not cover the matrix.

The matrix is prevented from coming into contact with the atmosphere by placing the cover layer 12 thereon, which seals the matrix. The cover layer is also formed of a laminate having the same construction as the backing layer, i.e. an outer layer 28 made of a polyester, e.g. polyethylene terephthalate; an intermediate layer 30 made of a metallic foil, e.g. aluminium foil; and an inner layer 32 of an ionomer, e.g. Surlyn. The surface of the inner layer coming into contact with the pressure sensitive adhesive 26 on the backing member 10 is coated with a release layer 34 to permit easy removal of the cover layer.

To apply the drug to the patient, the cover layer is peeled off. The exposed matrix is then taped onto a suitable portion of the patient's body, e.g. arm or wrist, to allow the drug to diffuse thereinto.

In the embodiment wherein trinitroglycerol is dispersed in the polymeric diffusion matrix, the molten matrix is cast into cavities provided in the backing member. The matrix is permitted to cure for a short period (e.g. about 10 minutes to about one hour) and is sealed by placing the cover layer over the backing member.

The matrices of the present invention are illustrated in the following Examples. Since the Examples are for illustrative purposes, they should not be construed as limiting.

## EXAMPLE I

100 ml of water and 20 ml of glycerol are mixed together and heated to about 50°C. 8 gm of polyvinylalcohol (molecular weight 126,000, 100 % hydrolyzed) is slowly added while the preparation is undergoing rapid agitation. After the polyvinylalcohol is uniformly dispersed, 5 gm of polyvinylpyrrolidone (molecular weight 40,000) is added with continued stirring. The preparation is then heated to about 95°C until solution is effected. At this point, the preparation may be cast onto a flat preparation so that it can harden.

The homogenous mixture is poured onto a stainless steel plate resulting in an uncured burn matrix having a thickness of about 3 to 4 mm. The burn matrix is cured by letting water evaporate for about 24 hours, leaving a cured burn matrix having a thickness of about 1 to about 2 mm.

The burn matrix has the following compositions:

| Ingredients | % by weight uncured | cured |
|---|---|---|
| glycerol | 17.9 | 34.6 |
| polyvinylalcohol | 5.8 | 11.3 |
| polyvinylpyrrolidone | 3.6 | 7.0 |
| water | 72.7 | 47.0 |

EXAMPLE II

A piece of the cured burn matrix of Example I is placed on a test system of 10% aqueous gelatin cast into a petri plate, to serve as a model for testing the burn matrix. On this model, it was found that the burn matrix of the present invention does not appreciably swell but does permit a small amount of water evaporation and further permits the exchange of some gases by diffusion processes across the perpendicular dimension of the burn matrix. The burn matrix of the present invention retards the loss of water vapour from a 10% gelatin preparation by approximately a factor of 10.

EXAMPLE III

A one inch (2.5 cm) square piece of the cured burn matrix of Example I is used as a model for preparing a burn matrix with water-soluble medicinal additives. Painted onto one side of the burn matrix of Example I is 10 mg of Xylocaine. After painting the Xylocaine onto the burn matrix, the burn matrix is permitted to stand for about 4 hours, resulting in a burn matrix having the Xylocaine diffused therein.

EXAMPLE IV

In place of the Xylocaine of Example III, 30 mg of Penicillin VK is applied to the burn matrix, resulting in a burn matrix having antibiotic properties over the 24 hour period desired for the life of the burn matrix.

EXAMPLE V

Example III is repeated, except that in addition to the Xylocaine, there is also simultaneously painted onto the burn matrix 30 mg of Penicillin VK. The resulting burn matrix provides both antibiotic protection against infection and relief from pain over an extended period, due to the slow release of the

- 22 -

xylocaine over a prolonged period.

EXAMPLE VI

The procedure of Example I is repeated, with the following variations: 18 ml instead of 20 ml of glycerol are used. In addition, sufficient zinc sulphadiazine to make up 1 % by weight of the final cured burn matrix is suspended in 2 ml of glycerol. This suspension is added to the mixture of other ingredients as the last step prior to pouring onto the stainless steel plate.

The resulting cured burn matrix provides the additional advantage of protecting the burn victim over an extended period against Pseudomonas infection. In place of zinc sulphadiazine, silver sulphadiazine may be used.

EXAMPLE VII

The procedure of Example VI is repeated except that the zinc sulphadiazine is replaced by 20 mg of Cephalosporin, resulting in a burn matrix having antibiotic properties.

- 1 -

CLAIMS:

1. A burn matrix comprising from about 2 to about 60 % of glycerol, from about 2 to about 15 % of polyvinylalcohol, from about 2 to about 10 % of a water-soluble polymer with hydration sites which in combination with the remaining ingredients yield a matrix capable of sustained release of a drug dispersed therein, and the balance water, the percentages being by weight.

2. A burn matrix as claimed in claim 1, wherein the matrix is cured and comprises from about 2 to about 55 % of glycerol, from about 4 to about 30 % of polyvinylalcohol, from about 2 to about 20 % of the water-soluble polymer having hydration sites, and the balance water.

3. A burn matrix as claimed in claim 1 or 2, wherein the water-soluble polymer is polyvinylpyrrolidone, agar, agarose or a water-soluble cellulose derivative.

4. A burn matrix as claimed in any one of claims 1 to 3, wherein the polyvinylalcohol has a molecular weight of from about 100,000 to about 150,000, and the polyvinylpyrrolidone, when present as the water-soluble polymer, has a molecular weight of from about 20,000 to about 60,000.

5. A burn matrix as claimed in any one of claims 1 to 4, having dispersed therein a therapeutically effective amount of at least one drug suitable for transdermal delivery to a patient, or containing or having deposited on the surface thereof a therapeutically effective amount of at least one drug suitable for topical delivery to a patient.

6. A burn matrix as claimed in claim 5, wherein the topical drug is an antibiotic, a local anesthetic, an analgesic, a fungicide, a bactericide, or an anti-micoplasma.

7.    A burn treatment device comprising a burn matrix as claimed in any one of claims 1 to 6, and means for securing said matrix to the skin of a patient.

8.    A method of making a burn matrix comprising, in an uncured state and on a weight basis, from about 2 to about 60 % of glycerol, from about 2 to about 15 % of polyvinylalcohol, from about 2 to about 10 % of a water-soluble polymer having hydration sites, and the balance water, which method comprises mixing the constituents, heating the mixture and casting the mixture to form the matrix.

9.    A method as claimed in claim 8, comprising the following steps:

(a) mixing the glycerol with water;

(b) dissolving the polyvinylalcohol and the water-soluble polymer having hydration sites in the mixture of (a) by stirring and heating to from about 90 to about 95°C; and

(c) casting the mixture to form sheets of the diffusion matrix.

10.    A method as claimed in claim 9, including the additional step of curing the matrix obtained in (c) to produce a cured matrix comprising, on a weight basis, from about 2 to about 55 % of glycerol, from about 4 to about 30 % of polyvinylalcohol, from about 2 to about 20 % of the water-soluble polymer having hydration sites, and the balance water.

11.    A method as claimed in any one of claims 8 to 10, wherein a therapeutically effective amount of at least one drug suitable for transdermal or topical application to a patient is added to and dispersed in the mixture or deposited on the surface of the matrix.

0040862

- 3 -

12. A matrix as defined in any of claims 1 to 6, or when made by a method as claimed in any of claims 8 to 11, or a device as claimed in claim 7, for use in the treatment of burns.

- 1 -

(Austria)

CLAIMS:

1. A method of making a burn matrix comprising, in an uncured state and on a weight basis, from about 2 to about 60 % of glycerol, from about 2 to about 15 % of polyvinylalcohol, from about 2 to about 10 % of a water-soluble polymer having hydration sites, and the balance water, which method comprises mixing the constituents heating the mixture, and casting the mixture to form the diffusion matrix.

2. A method as claimed in claim 1, comprising the following steps:

   (a) mixing the glycerol with water;

   (b) dissolving the polyvinylalcohol and the water-soluble polymer having hydration sites in the mixture of (a) by stirring and heating to from about 90 to about 95°C; and

   (c) casting the mixture to form sheets of the diffusion matrix.

3. A method as claimed in claim 1 or 2, wherein the water-soluble polymer is agar, agarose, polyvinylpyrrolidone or a water-soluble cellulose derivative.

4. A method as claimed in claim 3, wherein the water-soluble polymer is polyvinylpyrrolidone and including the additional step of curing the matrix obtained in (c) to produce a cured matrix comprising, on a weight basis, from about 2 to about 55 % of glycerol, from about 4 to about 30 % of polyvinylalcohol, from about 2 to about 20 % of polyvinylpyrrolidone, and the balance water.

5. A method as claimed in any one of claims 2 to 4, wherein a therapeutically effective amount of at least one drug suitable for transdermal or topical application to a patient is added to and dispersed in the mixture of (b).

0040862

- 2 -
(Austria)

6.　A method as claimed in any one of claims 1 to 4, including the additional step of depositing on the surface of the matrix a therapeutically effective amount of at least one drug suitable for transdermal or topical application to a patient.

7.　A burn matrix comprising from about 2 to about 60 % of glycerol, from about 2 to about 15 % of polyvinylalcohol, from about 2 to about 10 % of a water-soluble polymer with hydration sites which in combination with the remaining ingredients yield a matrix capable of sustained release of a drug dispersed therein, and the balance water, the percentages being by weight, for use in the treatment of burns.

8.　A burn matrix as claimed in claim 7, wherein the matrix is cured and comprises from about 2 to about 55 % of glycerol, from about 4 to about 30 % of polyvinylalcohol, from about 2 to about 20 % of the water-soluble polymer having hydration sites and the balance water.

9.　A burn matrix as claimed in claims 7 or 8, wherein the water-soluble polymer is polyvinylpyrrolidone, agar, agarose or a water-soluble cellulose derivative.

10.　A burn matrix as claimed in any one of claims 7 to 9, wherein the polyvinylalcohol has a molecular weight of from about 100,000 to about 150,000, and the polyvinylpyrrolidone, when present as the water-soluble polymer, has a molecular weight of from about 20,000 to about 60,000.

11.　A burn matrix as claimed in any one of claims 7 to 10, having dispersed therein a therapeutically effective amount of at least one drug suitable for transdermal delivery to a patient, or containing or having deposited on the surface thereof a therapeutically effective amount of at least one drug suitable for topical delivery to a patient.

- 3 -

(Austria)

12.　A burn matrix as claimed in claim 11, wherein the topical drug is an antibiotic, a local anesthetic, an analgesic, a fungicide, a bactericide, or an anti-micoplasma.

0040862

Fig.1

Fig.2

## DOCUMENTS CONSIDERED TO BE RELEVANT

| Category | Citation of document with indication, where appropriate, of relevant passages | Relevant to claim |
|---|---|---|
| X | GB - A - 1 108 837 (ASTRA PHARMA-CEUTICAL PRODUCTS)<br><br>* Page 1, lines 13-15; page 2, lines 5-37, 63-127; page 3, lines 66-79; examples 13,14; page 6, lines 51-124; claims 1,3,7,8 *<br><br>--- | 1-3,5, 6 |
| A | US - A - 3 598 123 (A. ZAFFARONI)<br><br>* Figures; column 3, line 48 to column 4, line 27; column 5, lines 6-10; column 6, lines 1-13; claims * | 1,7 |

----------

**CLASSIFICATION OF THE APPLICATION (Int. Cl.)**

A 61 K 9/70
A 61 L 15/01
A 61 L 15/03

**TECHNICAL FIELDS SEARCHED (Int. Cl.³)**

A 61 K 9/22
9/26
9/70
A 61 L 9/04
15/01
15/03
15/06

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 14.09.1981 | WILLEKENS |

EPO Form 1503.1   06.78